# EUROPEAN PATENT SPECIFICATION

(11) **EP 1 152 724 B1**
(45) Date of publication and mention of the grant of the patent: **18.06.2003**
(21) Application number: 99908270.4
(22) Date of filing: 19.02.1999
(51) Int. Cl.: A61F 13/15

(54) **APPARATUS FOR MANUFACTURING HYGIENIC ARTICLES HAVING A HORIZONTAL CIRCULAR DRUM**
VORRICHTUNG MIT EINER KREISFÖRMIGEN HORIZONTALEN TROMMEL ZUR HERSTELLUNG VON HYGIENEARTIKELN
DISPOSITIF SERVANT A FABRIQUER DES ARTICLES HYGIENIQUES ET POSSEDANT UN TAMBOUR HORIZONTAL CIRCULAIRE

(43) Date of publication of application: 14.11.2001
(73) Proprietor: The Procter & Gamble Company, Cincinnati, Ohio 45202 (US)
(72) Inventor: SCHMITZ, Christoph, Johann, D-53881 Euskirchen-Stotzheim (DE)
(74) Representative: Kremer, Véronique
(86) International application number: US9903596
(87) International publication number: WO00048542

(56) References cited:
- EP-A- 0 589 859
- WO-A-98/00356
- FR-A- 2 215 178

## Description

### FIELD OF THE INVENTION

The present invention relates to an apparatus for the manufacture of a series of discrete hygienic articles and to a process for manufacturing a series of discrete hygienic articles.

### BACKGROUND

Hygienic articles such as absorbent pads, diapers, training pants, adult incontinence devices, sanitary napkins, bed pads, wound dressings and the like are well known in the art. These articles, in particular the disposable hygienic articles, have become a mass market. Hence, these articles have to be manufactured at a high speed and at a low cost.

Generally, such hygienic articles are manufactured by providing a continuous base material such as for example the backsheet or the topsheet, joining other elements of the article to the base material at regular intervals, and finally cutting the continuous web of articles into discrete articles. Such a process and a suitable apparatus are disclosed for example in EP-A-589859.

Conventional apparatus such as the one disclosed in EP-A-589859 are arranged in an essentially linear fashion. The drive means transporting the webs are typically arranged on one side of the linear web path whereas the operators access the line from the opposite side. The manipulations means are arranged along the line.

In FR-A-2 215 178 a cylindrical drum is used as manipulation means for cutting a continuous web into transversal sections. Also in WO-A-98/00356 a cylindrical drum is used for transversally positioning <-> and placing material components on absorbent articles transported on a conveyor belt.

Whilst being used widely within the industry, these apparatus have inherent restrictions such as limited space that is available for the installation of manipulation means. The obvious solution of extending the length of the line significantly increases the complexity of the apparatus since web tension control means and driving of the line require much more effort on longer manufacturing lines.

### SUMMARY OF THE INVENTION

The present invention provides an apparatus for manufacturing hygienic articles comprising a supply means supplying hygienic articles or hygienic article precursors, a plurality of manipulation means for manipulating the hygienic articles or the hygienic article precursors, and a reception means receiving hygienic articles or hygienic article precursors. A segment of the manufacturing path is positioned intermediate the supply means and the reception means whilst the manipulation means are arranged to manipulate the hygienic articles or the hygienic article precursors on said manufacturing path segment. The apparatus of the present invention is characterized in that it comprises a cylindrical drum having a vertical axis of rotation. The supply means is arranged to supply the hygienic articles or the hygienic article precursors to the outer surface of said cylindrical drum. At least half of the manufacturing path segment is positioned on the outer surface of said cylindrical drum. The reception means is arranged to receive the hygienic articles or the hygienic article precursors from the outer surface of said cylindrical drum.

The present invention further provides a process for manufacturing hygienic articles. The process comprises the steps of
- supplying hygienic articles or hygienic article precursors
- transporting said hygienic articles or said hygienic article precursors along a manufacturing path, at least half of said manufacturing path being positioned on a surface corresponding to the outer surface of a cylinder having a vertical axis of symmetry,
- carrying out a plurality of manipulation steps on said hygienic articles or hygienic article precursors while said hygienic articles or said hygienic article precursors are being transported on said manufacturing path segment,
- receiving hygienic articles or hygienic article precursors from said manipulation path segment.

### BRIEF DESCRIPTION OF THE DRAWINGS

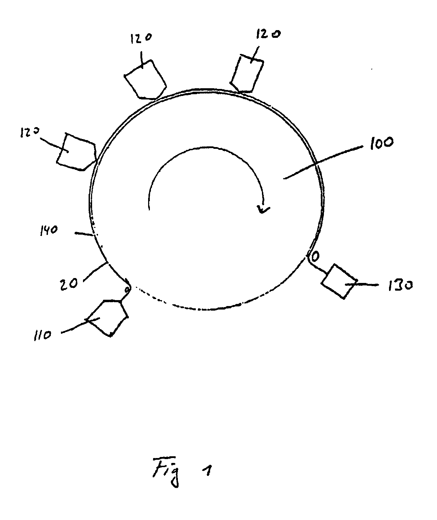

FIG.1 shows a schematic top view of an exemplary embodiment of the apparatus of the present invention.

### DETAILED DESCRIPTION OF THE INVENTION

The present invention provides an apparatus for manufacturing hygienic articles. In one embodiment of the apparatus a web of hygienic article precursors is manipulated to be a web of hygienic articles which may be finally severed into discrete hygienic articles. For example, the web of hygienic article precursors may be manipulated by adding a web of hygienic article elements or by adding discrete hygienic article elements to the web. In an alternative embodiment of the apparatus of the present invention, discrete hygienic article precursors are manipulated to become discrete hygienic articles.

The term "hygienic article" as used herein refers to articles which are intended to be used in contact with or in close proximity to the skin of the user. Hygienic articles include absorbent articles such as absorbent pads, diapers, training pants, adult incontinence devices, sanitary napkins, bed pads, and the like. In addition, hygienic articles comprise articles of low or zero absorbency such as wound dressings, clothing (in particular underwear), and the like. Hygienic articles of the present invention may be intended for multiple use or they may be disposable. The term "disposable" is used herein to describe hygienic articles which are not intended to be laundered or otherwise restored or reused as a hygienic article after a single use.

Typically, hygienic articles are manufactured from essentially flat elements and hence the hygienic articles essentially have a flat configuration unless they are given a three-dimensional shape by contraction means such as elastics or the like. The term "flat" as used herein refers to articles, precursors or elements which have a longitudinal dimension, a transverse dimension, and a thickness dimension substantially smaller than the longitudinal dimension and the substantially smaller than the transverse dimension.

The term "precursor" of an article refers to that partially assembled precursor of an article that comprises that element of the article which has been supplied to the process first.

The term "element" of an article as used herein refers to any part of the article that is joined to the rest of the article.

Preferably, the apparatus of the present invention can manufacture hygienic articles in a continuous process at an output rate of at least 10 articles per minute, more preferably more than 20 articles per minute, even more preferably more than 50 articles per, even more preferably more than 100 articles per minute, most preferably more than 300 articles per minute.

In FIG.1, an schematic top view of an exemplary embodiment of the apparatus of the present invention is shown. The apparatus comprises a cylindrical drum 100, a supply means 110, a plurality of manipulation means 120, and a reception means 130. A segment of the manufacturing path 140 of the hygienic articles 20 runs from the supply means 110 onto the outer surface of the cylindrical drum 100 to the reception means 130. During manufacture, the cylindrical drum is rotated around its central axis of symmetry according to the arrow in FIG. 1. The manipulation means 120 manipulates the hygienic articles 20 while they are positioned on the outer surface of the cylindrical drum 100.

The term "cylindrical drum" as used herein refers to an element of an apparatus that has a substantially cylindrical shape being characterized by a radial dimension and a thickness dimension, the thickness dimension usually being substantially smaller than the radial dimension. Typically, a cylindrical drum is rotated around its central axis of symmetry. The term "cylindrical drums" include drums having a circular circumference and drums having a polygonal circumference. A polygonal drum preferably has more than 8 outer surface segments, more preferably more than 12 outer surface segments, most preferably more than 20 outer surface segments. The term "cylindrical drums" further comprises drums in which the radial dimension varies along the thickness dimension, the variation being small compared to the radial dimension.

The term "outer surface" of a cylindrical drum refers to that surface of the cylindrical drum that is perpendicular to the radial dimension of the cylindrical drum.

The term "supply means" as used herein refers to a device that is capable of supplying a web of hygienic articles, a web of hygienic article precursors, discrete hygienic articles, or discrete hygienic article precursors.

The term "manipulation means" refers to any part of an apparatus for the manufacture of articles that performs an action on a hygienic article, a precursor of the article, or any element of the article. Such actions include but are not limited to, adding or removing of elements, change of physical or chemical state such as cutting, folding, heating, compressing and the like.

The term "reception means" as used herein refers to a device that is capable of receiving a web of hygienic articles, a web of hygienic article precursors, a web of hygienic article elements, discrete hygienic articles, discrete hygienic article precursors, or discrete hygienic article elements.

The term "manufacturing path" as used herein refers to the path of the articles or its precursors through the apparatus.

The term "manufacturing path segment" as used herein refers to a segment of the manufacturing path between a first manipulation means and a second manipulation means of the apparatus. In this context, the first manipulation means can be the supply means and the second manipulation means can be the reception means. Typically, the first and second manipulation means are spatially separated from each other such that the article or its precursor needs to be transported from the first to the second manipulation means.

In one embodiment of the apparatus of the present invention, the supply means of the present invention is an unwind for a roll stock material such as the backsheet or the topsheet of an hygienic article. In another embodiment, the supply means is a source of fibers that are applied to the outer surface of the cylindrical drum where they may held for example by means of a vacuum. In another embodiment, the supply means may supply a discrete hygienic article precursor or element to the outer surface of the cylindrical drum.

In some embodiments of the apparatus of the present invention, the hygienic article, precursor or element supplied to the outer surface of the cylindrical drum may comprise more than one element, i.e. one or more manipulation means are arranged upstream of the supply means.

The cylindrical drum of the apparatus has a vertical axis of symmetry around which the drum is rotating. The speed of rotation of the drum is chosen such that the linear speed of the outer surface is substantially equal to the average linear speed of the hygienic articles or hygienic article precursors along the manufacturing path segment.

While the hygienic articles, precursors, or elements are positioned on the outer surface of the cylindrical drum, their thickness dimension is aligned horizontally. Hence, the major surfaces of the hygienic article, precursors or elements are oriented vertically. Thus, dust particles and other contaminations can not fall onto the major surfaces of the hygienic articles, precursors, or elements. Moreover, dust particles already sitting on the major surfaces are pulled away from the surface by gravity and centrifugal forces.

The radius of the cylindrical drum of the present invention preferably is between 100 mm and 10000mm, more preferably between 200 mm and 5000 mm, even more preferably between 400mm and 2500mm, most preferably between 500 mm and 2000 mm.

The cylindrical drum layout of the apparatus of the present invention offers the advantage to drive a long manufacturing path segment with a single drive means controlling the rotation of the cylindrical drum. On conventional linear manufacturing lines, a plurality of drive means connected via shafts, belts, and the like had to be arranged along the line.

In one embodiment of the apparatus of the present invention, a continuous web of hygienic articles, a continuous web of hygienic article precursors, or a continuous web of hygienic article elements is supplied to the outer surface of the cylindrical drum. The web may be placed on the outer surface of the cylindrical drum until it is received by the reception means. The tension of a web at least partially supported by the outer surface of a cylindrical drum is much easier to control than the tension of a web on a linear manufacturing line.

In the present invention, the manipulation means are arranged such that they manipulate the hygienic articles, precursors, or elements on the outer surface of the cylindrical drum, i.e. the manipulation means are positioned radially outside of the outer surface of the cylindrical drum. In this arrangement, the space that is available for the installation of the manipulation means resembles a sector of a cylinder. On a conventional linear hygienic article manufacturing line, the available space corresponds to a rectangular slab having the width of the inner edge of the corresponding cylinder on an apparatus according to the present invention. Hence, by arranging the manipulation means around the outer surface of a cylindrical drum, an increase of the space available to manipulation means relative to the length of the line is obtained.

Examples of manipulation means suitable for the apparatus of the present invention are well known in the art and are described, for example, in EP-A-589859.

In some embodiments of the apparatus of the present invention, at least one manipulation means temporarily removes the hygienic article or hygienic article precursor from the outer surface of the cylindrical drum, performs the manipulation, and then restores the article or precursor on the outer surface again.

In some embodiments of the apparatus of the present invention, the manipulation means or at least parts thereof may temporarily move along the outer surface of the cylindrical drum so as to temporarily change the speed of the manipulation means or the part thereof relative to the outer surface. Such a change in speed may offer advantages for some manipulation steps.

In one embodiment of the apparatus of the present invention, the reception means is a packaging means which receives hygienic articles from the outer surface of the cylindrical drum and subsequently packages or stores them. In another embodiment of the present invention, the reception means is a cutting means which receives a web of hygienic articles from the outer surface of the cylindrical drum and subsequently severs them into discrete hygienic articles. In another embodiment, the reception means receives a hygienic article, a hygienic article precursor, or an hygienic article element from the outer surface of the cylindrical drum and subsequently transports them to additional manipulation for further manufacturing steps.

In another embodiment of the apparatus of the present invention, the receiving means is a second cylindrical drum also having a vertical axis of rotation. In this case, the apparatus comprises a second manufacturing path segment, at least a part of which is arranged on the outer surface the second cylindrical drum. Further manipulation means may be arranged around the outer surface of said second cylindrical drum. Such an arrangement of two cylindrical drums allows to easily flip the orientation of the hygienic articles, precursors, or elements relative to the outer surfaces of the drums.

In one embodiment of the apparatus of the present invention, the cylindrical drum comprises movable carrier elements on its outer surface which can be moved along the circumference of the cylindrical drum, i.e. along the manufacturing path segment positioned on the outer surface of the drum. With these carrier it is possible to temporarily change the speed of transportation of parts of a web of articles or of discrete particles along the manufacturing path segment.

It is another aspect of the present invention to provide a process for manufacturing hygienic articles such as absorbent articles and in particular disposable absorbent articles.

In one embodiment of the process of the present invention, a web of hygienic articles or hygienic articles precursors is supplied to the process. In another embodiment of the process of the present invention, discrete hygienic articles or discrete hygienic article precursors are supplied to the process.

Subsequently, the hygienic articles or hygienic are transported on a manufacturing path segment at least half of which is arranged on a surface that corresponds to the outer surface of a cylinder having a vertical axis of symmetry. A plurality of manipulation steps is carried out while the hygienic articles or hygienic article precursors are transported on the manufacturing path segment. In one embodiment of the process of the present invention, the hygienic articles or hygienic articles precursors are temporarily removed from the surface corresponding to the outer surface of a cylinder to carry out one or more manipulation steps. Subsequently, the hygienic articles or hygienic article precursors are placed back on the surface corresponding to the outer surface of a cylinder.

At the end of the manufacturing path segment arranged on a surface corresponding to the outer surface of a cylinder, the hygienic articles or hygienic article precursors are received from the manufacturing path segment. The receiving step may be followed by a severing step to convert a web of articles or precursors into discrete articles or precursor, a packaging step to package one or more articles or precursors at a time, or any other step that is required for the further manufacture of the hygienic article or hygienic article precursor.

In one embodiment of the apparatus of the present invention, more than one manufacturing path segment runs in parallel on the outer surface of the cylindrical drum. The hygienic articles on these parallel path segments might be manipulated individually or as a group by the manipulation means. Similarly, they may originate from one or more supply means and lead to one or more reception means.

## Claims

1. An apparatus for manufacturing hygienic articles, said apparatus comprising
- supply means (140) supplying hygienic articles or hygienic article precursors
- a plurality of manipulation means (120) for manipulating hygienic articles or hygienic article precursors,
- reception means (130) receiving hygienic articles or hygienic article precursors, said apparatus having a manufacturing path segment (140) intermediate said supply means and said reception means, said manipulation means being arranged to manipulate said hygienic articles or said hygienic article precursors on said manufacturing path segment, **characterized in that** said apparatus comprises a cylindrical drum (100) having a vertical axis of rotation, said supply means (110) supplies hygienic articles or hygienic article precursors to the outer surface of said cylindrical drum (100), at least half of said manufacturing path segment (140) is positioned on the outer surface of said cylindrical drum, said reception means (130) receives hygienic articles or hygienic article precursors from the outer surface of said cylindrical drum.

2. An apparatus for manufacturing hygienic articles according to Claim 1, wherein the rotational speed of said cylindrical drum is such that the linear velocity of outer surface of said cylindrical drum is substantially equal to the average linear velocity of said hygienic articles or said hygienic article precursors along said manufacturing path segment.

3. An apparatus for manufacturing hygienic articles according to Claim 1, wherein said apparatus comprises
- a second cylindrical drum having a substantially vertical axis,
- a plurality of second manipulation means positioned around the outer surface of said second cylindrical drum, said apparatus has a second manufacturing path segment, said second manufacturing path segment being positioned substantially on a part of the outer surface of said second cylindrical drum and said second manipulation means manipulating said hygienic articles or their precursors on said second manufacturing path segment.

4. An apparatus for manufacturing hygienic articles according to Claim 1 wherein said supply means supplies discrete hygienic articles or discrete hygienic article precursors.

5. An apparatus for manufacturing hygienic articles according to Claim 1 wherein said supply means supplies a continuous web of hygienic articles or a continuous web of hygienic article precursors.

6. An apparatus for manufacturing hygienic articles according to Claim 1, wherein said hygienic articles are absorbent articles.

7. An apparatus for manufacturing hygienic articles according to Claim 3, wherein said absorbent articles are disposable absorbent articles.

8. A process for manufacturing hygienic articles, said process comprising the steps of
- supplying hygienic articles or hygienic article precursors
- transporting said hygienic articles or said hygienic article precursors along a manufacturing path, at least half of said manufacturing path being positioned on a surface corresponding to the outer surface of a cylinder having a vertical axis of symmetry,
- carrying out a plurality of manipulation steps on said hygienic articles or hygienic article precursors while said hygienic articles or said hygienic article precursors are being transported on said manufacturing path segment,
- receiving hygienic articles or hygienic article precursors from said manipulation path segment.

9. A process for manufacturing hygienic articles according to Claim 8 wherein said hygienic articles are absorbent articles.

10. A process for manufacturing hygienic articles according to Claim 9 wherein said hygienic articles are disposable absorbent articles.

## Patentansprüche

1. Vorrichtung zur Herstellung von Hygieneartikeln, wobei die Vorrichtung umfasst
- ein Zuführmittel (110), das Hygieneartikel oder Hygieneartikel-Vorprodukte zuführt,
- eine Vielzahl von Bearbeitungsmitteln (120) zum Bearbeiten von Hygieneartikeln oder Hygieneartikel-Vorprodukten,
- ein Aufnahmemittel (130), das Hygieneartikel oder Hygieneartikel-Vorprodukte aufnimmt,
wobei die Vorrichtung einen Herstellungs-Bahnabschnitt (140) zwischen dem Zuführmittel und dem Aufnahmemittel aufweist, wobei die Bearbeitungsmittel angeordnet sind, um die Hygieneartikel oder die Hygieneartikel-Vorprodukte auf dem Herstellungs-Bahnabschnitt zu bearbeiten, **dadurch gekennzeichnet, dass** die Vorrichtung eine zylindrische Walze (100) mit einer vertikalen Rotationsachse aufweist,
wobei das Zuführmittel (110) Hygieneartikel oder Hygieneartikel-Vorprodukte zu der Außenfläche der zylindrischen Walze (100) zuführt, wobei mindestens die Hälfte des Herstellungs-Bahnabschnittes (140) auf der Außenfläche der zylindrischen Walze vorgesehen ist,
wobei das Aufnahmemittel (130) Hygieneartikel oder Hygieneartikel-Vorprodukte von der Außenfläche der zylindrischen Walze aufnimmt.

2. Vorrichtung zur Herstellung von Hygieneartikeln gemäß Anspruch 1,
wobei die Rotationsgeschwindigkeit der zylindrischen Walze derart ist, dass die lineare Geschwindigkeit der Außenfläche der zylindrischen Walze im Wesentlichen gleich der durchschnittlichen linearen Geschwindigkeit der Hygieneartikel oder der Hygieneartikel-Vorprodukte entlang dem Herstellungs-Bahnabschnitt ist.

3. Vorrichtung zur Herstellung von Hygieneartikeln gemäß Anspruch 1,
wobei die Vorrichtung umfasst
- eine zweite zylindrische Walze mit einer im Wesentlichen vertikalen Achse,
- eine Vielzahl von zweiten Bearbeitungsmitteln, die um die Außenfläche der zweiten zylindrischen Walze angeordnet sind, wobei die Vorrichtung einen zweiten Herstellungs-Bahnabschnitt aufweist,
wobei der zweite Herstellungs-Bahnabschnitt im Wesentlichen auf einem Teilstück der Außenfläche der zweiten zylindrischen Walze angeordnet ist und die zweiten Bearbeitungsmittel die Hygieneartikel oder ihre Vorprodukte auf dem zweiten Herstellungs-Bahnabschnitt bearbeiten.

4. Vorrichtung zur Herstellung von Hygieneartikeln gemäß Anspruch 1,
wobei das Zuführmittel einzelne Hygieneartikel oder einzelne Hygieneartikel-Vorprodukte zuführt.

5. Vorrichtung zur Herstellung von Hygieneartikeln gemäß Anspruch 1,
wobei das Zuführmittel eine kontinuierliche Bahn von Hygieneartikeln oder eine kontinuierliche Bahn von Hygieneartikel-Vorprodukten zuführt.

6. Vorrichtung zur Herstellung von Hygieneartikeln gemäß Anspruch 1,
wobei die Hygieneartikel absorbierende Artikel sind.

7. Vorrichtung zur Herstellung von Hygieneartikeln gemäß Anspruch 3,
wobei die absorbierenden Artikel absorbierende Wegwerf-Artikel sind.

8. Verfahren zur Herstellung von Hygieneartikeln, wobei das Verfahren die Schritte umfasst:
- Zuführen von Hygieneartikeln oder Hygieneartikel-Vorprodukten,
- Fördern der Hygieneartikel oder der Hygieneartikel-Vorprodukte entlang einer Herstellungs-Bahn, wobei mindestens die Hälfte der Herstellungs-Bahn auf einer Oberfläche vorgesehen ist, die der Außenfläche eines Zylinders mit einer vertikalen Symmetrieachse entspricht,
- Ausführen einer Vielzahl von Bearbeitungsschritten an den Hygieneartikeln oder den Hygieneartikel-Vorprodukten, während die Hygieneartikel oder die Hygieneartikel-Vorprodukte auf dem Herstellungs-Bahnabschnitt befördert werden,
- Aufnehmen von Hygieneartikeln oder Hygieneartikel-Vorprodukten aus dem Herstellungs-Bahnabschnitt.

9. Verfahren zur Herstellung von Hygieneartikeln gemäß Anspruch 8,
wobei die Hygieneartikel absorbierende Artikel sind.

10. Verfahren zur Herstellung von Hygieneartikeln gemäß Anspruch 9,
wobei die Hygieneartikel absorbierende Wegwerf-Artikel sind.

## Revendications

1. Dispositif pour fabriquer des articles hygiéniques, ledit dispositif comprenant :
- des moyens d'alimentation (110) fournissant des articles hygiéniques ou des ébauches d'articles hygiéniques,
- une pluralité de moyens de manipulation (120) destinés à manipuler les articles hygiéniques ou les ébauches d'articles hygiéniques,
- des moyens de réception (130) recevant les articles hygiéniques ou les ébauches d'articles hygiéniques,
ledit dispositif présentant un segment (140) de trajet de fabrication entre lesdits moyens d'alimentation et lesdits moyens de réception, lesdits moyens de manipulation étant agencés pour manipuler lesdits articles hygiéniques ou lesdites ébauches d'articles hygiéniques sur ledit segment de trajet de fabrication, **caractérisé en ce que** ledit dispositif comprend un tambour cylindrique (100) ayant un axe vertical de rotation, lesdits moyens d'alimentation (110) fournissent les articles hygiéniques ou les ébauches d'articles hygiéniques à la surface extérieure dudit tambour cylindrique (100), au moins la moitié dudit segment (140) de trajet de fabrication est placée sur la surface extérieure dudit tambour cylindrique, lesdits moyens de réception (130) reçoivent les articles hygiéniques ou les ébauches d'articles hygiéniques provenant de la surface extérieure dudit tambour cylindrique.

2. Dispositif pour fabriquer des articles hygiéniques selon la revendication 1, dans lequel la vitesse de rotation dudit tambour cylindrique est telle que la vitesse linéaire de la surface extérieure dudit tambour cylindrique est sensiblement égale à la vitesse linéaire moyenne desdits articles hygiéniques ou desdites ébauches d'articles hygiéniques le long dudit segment de trajet de fabrication.

3. Dispositif pour fabriquer des articles hygiéniques selon la revendication 1, dans lequel ledit dispositif comprend :
- un deuxième tambour cylindrique ayant un axe essentiellement vertical,
- une pluralité de deuxièmes moyens de manipulation placés autour de la surface extérieure dudit deuxième tambour cylindrique, ledit dispositif présente un deuxième segment de trajet de fabrication, ledit deuxième segment de trajet de fabrication étant placé sensiblement sur une partie de la surface extérieure dudit deuxième tambour cylindrique, et lesdits deuxièmes moyens de manipulation manipulant lesdits articles hygiéniques ou leurs ébauches sur ledit deuxième segment de trajet de fabrication.

4. Dispositif pour fabriquer des articles hygiéniques selon la revendication 1, dans lequel lesdits moyens d'alimentation fournissent des articles hygiéniques distincts ou des ébauches distinctes d'articles hygiéniques.

5. Dispositif pour fabriquer des articles hygiéniques selon la revendication 1, dans lequel lesdits moyens d'alimentation fournissent une nappe continue d'articles hygiéniques, ou une nappe continue d'ébauches d'articles hygiéniques.

6. Dispositif pour fabriquer des articles hygiéniques selon la revendication 1, dans lequel lesdits articles hygiéniques sont des articles absorbants.

7. Dispositif pour fabriquer des articles hygiéniques selon la revendication 6, dans lequel lesdits articles absorbants sont des articles absorbants jetables.

8. Procédé pour fabriquer des articles hygiéniques, ledit procédé comprenant les étapes consistant à :
- alimenter les articles hygiéniques ou les ébauches d'articles hygiéniques,
- transporter lesdits articles hygiéniques ou lesdites ébauches d'articles hygiéniques le long d'un trajet de fabrication, au moins la moitié dudit trajet de fabrication étant placée sur une surface correspondant à la surface extérieure d'un cylindre ayant un axe vertical de symétrie,
- effectuer plusieurs étapes de manipulation sur lesdits articles hygiéniques ou lesdites ébauches d'articles hygiéniques alors que lesdits articles hygiéniques ou lesdites ébauches d'articles hygiéniques sont trarisporté(e)s sur ledit segment de trajet de fabrication,
- recevoir les articles hygiéniques ou les ébauches d'articles hygiéniques provenant dudit segment de trajet de fabrication.

9. Procédé pour fabriquer des articles hygiéniques selon la revendication 8, dans lequel lesdits articles hygiéniques sont des articles absorbants.

10. Procédé pour fabriquer des articles hygiéniques selon la revendication 9, dans lequel lesdits articles hygiéniques sont des articles absorbants jetables.
